# EUROPEAN PATENT APPLICATION

(11) **EP 4 461 287 A1**
(43) Date of publication of application: **13.11.2024**
(21) Application number: 23382441.6
(22) Date of filing: 11.05.2023
(51) Int. Cl.: A61K 8/11, A61K 8/64, A61K 8/73, A61K 8/85, A61Q 19/08

(54) **SKIN CARE COSMETIC COMPOSITION**

(71) Applicant: Creaciones Aromáticas Industriales, S.A., 08192 Sant Quirze del Vallès, Barcelona (ES)
(72) Inventor: Martinez, Denia, 08192 Sant Quirze del Vallès, Barcelona (ES); Bernal, Cristóbal, 08192 Sant Quirze del Vallès, Barcelona (ES); González, Pablo, 08192 Sant Quirze del Vallès, Barcelona (ES); Mourelle, Marisabel, 08192 Sant Quirze del Vallès, Barcelona (ES); Puigpinós, Albert, 08192 Sant Quirze del Vallès, Barcelona (ES)
(74) Representative: Clarke, Modet y Cía., S.L.

(57) **Abstract**

The present invention relates to a skin care cosmetic composition comprising polymeric nanocapsules encapsulating at least one cosmetic active ingredient, wherein the polymeric nanocapsules comprising an outer layer made of sodium hyaluronate or fucoidan, an intermediate layer made of a polysaccharide and an inner layer made of a biodegradable polymer; and wherein the cosmetic active ingredient is selected from the group consisting of acetylhexapeptide-8, sodium hyaluronate, fucoidan or a mixture thereof. The invention also relates to the manufacturing method of polymeric nanocapsules of the cosmetic composition and the use of the cosmetic composition for the treatment of signs of skin aging and/or skin imperfections.

## Description

### FIELD OF THE INVENTION

The present invention relates to a cosmetic composition. In particular, the present invention relates to a skin care cosmetic composition comprising polymeric nanocapsules encapsulating a cosmetic active ingredient for skin care. The invention also relates to the manufacturing method of polymeric nanocapsules of the cosmetic composition.

### BACKGROUND ART

Today, botulinum toxin, known under the trade name Botox^{®}, is undoubtedly one of the star products in the cosmetics market, due to its remarkable anti-wrinkle efficacy. This toxin is a neurotoxin produced by the bacterium *Clostridium botulinum* and its mechanism of action is based on its binding to the synaptic cell receptors of peripheral nerve of the muscle (SNAP-25) associated with the fibroblasts. This nerve is responsible for transmitting the stimulus by secreting neurotransmitters that trigger muscle contraction through the release of calcium (Ca²⁺) and sodium (Na⁺) ions. The binding of this toxin modifies the structure of the synaptic receptor, blocking the release of neurotransmitters and thus preventing muscle contraction, resulting in local relaxation of the muscles.

However, its high toxicity is a problem, as in uncontrolled doses it can cause botulism poisoning, which causes muscle paralysis that can affect respiratory function and lead to death. In addition, it is only administered intramuscularly and must be administered by a healthcare professional in an appropriate medical environment. This fact can be a limiting factor for the user, who in some cases tries to avoid invasive techniques and with the help of clinical centers.

Therefore, based on its mechanism of action, short-chain peptides have been developed that mimic the interaction of the toxin with the receptor. In addition, these peptides have a greater ability to cross the skin than botulinum toxin, allowing them to be used topically, and have less toxicity.

Acetylhexapeptide-8, commercially known under the registered trademark Argireline^{®}, is one of these peptides that mimics the action of botulinum toxin. It should be noted that its topical application shows evident limitations associated with the delivery of the peptide to the synaptic receptor target, as well as its integrity during the application process and/or its sensitivity to proteases or other degradation mechanisms integrated into the skin structure.

Another strategy implemented to solve the problem of wrinkles consists of the application of hyaluronic acid. As described in the literature, the application of hyaluronic acid to areas of skin that have lost firmness not only acts as a filler, but also causes a partial restoration of fibroblast functionality, increases collagen production, improves, and stimulates the functionality of the dermis and epidermis, and repairs disturbed cellular balances.

It also has a high-water retention capacity, that allows to increase the hydration of layers of the skin. Although its stimulation efficacy is a short-term and reversible effect because the intrinsic entropy of the system itself unbalances it again and causes all the previously described effects.

In addition, at the user level, it should be noted that for the correct functionality of hyaluronic acid its application must be carried out by a healthcare professional by intradermal injection.

In summary, both treatments above-mentioned (hyaluronic acid and botulinum toxin- Argireline ^{®}) represent two differentiated scientific approaches with proven efficacy for the treatment of wrinkles. Despite its application mechanism is a limiting factor for its massive use, since both require periodic applications and must be carried out by professionals in an appropriate medical environment, due to the intradermal/intramuscular application respectively.

At the same time as the anti-wrinkle cosmetic treatments described, it is important to highlight the appearance of non-invasive treatments through the application of topical creams. This procedure allows you to apply the product yourself, without medical supervision.

However, one of the main problems with topical application is an efficacy loss of active ingredient, because of its limited penetration into the skin, and for its physicochemical integrity compromised once deposited on the skin. In fact, the barrier structure of skin has mechanisms for the degradation of exogenous substances, such as proteases and other ones, so that in most cases the degradation of these ingredients is occurred, altering the required functionality.

One strategy is the encapsulation of the active ingredients.

For example, KR101865841B1 discloses a cosmetic composition for reducing skin wrinkles prepared by using a technology of a nano-capsule with excellent skin-permeability and stability wherein active ingredients permeate a deep portion of skin. The cosmetic composition comprises sodium hyaluronate as the main ingredient (5 to 15% by volume) and further comprises acetylhexapeptide-8 (4 to 6 vol% by volume), among other active compounds. In this case, the effect of the invention is achieved by the active ingredient of proline, lysine, caplet tripeptide-1 and lecithin, which are captured in the nanocapsules and is directly absorbed in the dermis layer, thereby maximizing the skin wrinkle and whitening effect.

However, one of the main problems of the current ingredients encapsulation system for topical application is that they are not suitable to allow their release at the site of action and without suffering prior degradation, so it remains an unresolved scientific and technological challenge. Consequently, there is no encapsulated product on the market that guarantees the maximum functionality of the active ingredients used to treat expression wrinkles by topical application.

Therefore, there is a need to find a suitable encapsulation of the active ingredients for cosmetic use that improves the penetration of the active ingredient into the skin and its protection against degradation by cellular enzymes, guaranteeing a better efficacy of the cosmetic product.

### SUMMARY OF INVENTION

In view of the problems of the prior art, the main object of the present invention is to provide a cosmetic composition with a significant effectiveness in the treatment and/or care of the skin.

The skin care cosmetic composition of the present invention comprises polymeric nanocapsules which have an excellent permeability and stability of penetration of the active ingredient deep into the skin.

In a first aspect, the present invention refers to a skin care cosmetic composition comprising polymeric nanocapsules encapsulating at least one cosmetic active ingredient, wherein the polymeric nanocapsules comprising an outer layer made of sodium hyaluronate or fucoidan, an intermediate layer made of a polysaccharide and an inner layer made of a biodegradable polymer; and wherein the cosmetic active ingredient is selected from the group consisting of acetylhexapeptide-8, sodium hyaluronate, fucoidan or a mixture thereof.

In a preferred embodiment, the polymeric nanocapsules comprises 50% to 68.5% by weight of biodegradable polymer, 30% to 45% by weight of active ingredient, 1% to 3% by weight of sodium hyaluronate or fucoidan in the outer layer and 0,5 to 2% by weight of polysaccharide, based on the total weight of the polymeric nanocapsules.

In another preferred embodiment, the polysaccharide is selected from chitosan or dextran.

In another preferred embodiment, the biodegradable polymer is selected from the group consisting of policaprolactone (PCL), poly(lactic-co-glycolic acid) (PLGA), poly-lactic acid (PLA) or carrageenan.

In another preferred embodiment, the polymeric nanocapsules comprises 50% to 68.5% by weight of polycaprolactone, 20% to 25% by weight of acetylhexapeptide-8, 10% to 20% by weight of sodium hyaluronate (as active ingredient), 1 % to 3% by weight of sodium hyaluronate (in the outer layer) and 0,5 to 2% by weight of chitosan, based on the total weight of the polymeric nanocapsules.

The cosmetic composition can be used in an anti-wrinkle treatment, skin care routine and other cosmetic applications.

Due to the structure of the nanocapsules and the combination of the ingredients, the efficacy of the active ingredients is superior to that of the free molecular versions of the compounds. Surprisingly, the anti-wrinkle action of the cosmetic composition is enhanced by a synergistic effect between the active ingredients and the nanocapsules.

The cosmetic composition of the present invention further comprises one or more cosmetically acceptable excipients.

Any excipients commonly used in the preparation of cosmetic preparations for use on the human skin may be employed in the present invention. Suitable excipients include but are not limited to ingredients that can influence organoleptic properties, penetration of the skin, and the bioavailability of the cosmetic active agent of the present invention. More specifically, they include liquids, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin, polyvinyl alcohol, xanthan gum, arabic gum among others.

The skin care cosmetic composition can be formulated as an emulsion, a cream, a lotion, a solution, a gel, an ointment or a serum.

In a second aspect of the invention, the present invention relates to method of manufacturing the polymeric nanocapsules of the cosmetic composition of the present invention and comprising the following steps:
a) adding a biodegradable polymer together with at least one cosmetic active ingredient wherein the cosmetic active ingredient is selected from the group consisting of acetylhexapeptide-8, sodium hyaluronate, fucoidan or a mixture thereof in the presence of an organic solvent to create a double emulsion;
b) stirring the emulsion obtained in step a) until complete evaporation of the organic solvent to obtain nanoparticles;
c) dropping the nanoparticles obtained in step b) into a solution of polysaccharide with continuous stirring; and
   adding the polysaccharide-coated nanoparticles obtained in step c) to a solution of sodium hyaluronate or fucoidan to obtain the polymeric nanocapsules.

In a preferred embodiment, the ratio of nanoparticles and the solution of polysaccharide in step c) is 1:2.

In another preferred embodiment, the ratio of polysaccharide-coated nanoparticles and the solution of sodium hyaluronate in step d) is 1:3.

In a preferred embodiment, the polysaccharide is selected from chitosan or dextran.

In another preferred embodiment, the biodegradable polymer is selected from the group consisting of policaprolactone (PCL), poly(lactic-co-glycolic acid) (PLGA), poly-lactic acid (PLA) or carrageenan.

In another preferred embodiment, the continuous stirring in step c) is for 2 h.

In a third aspect of the invention, the present invention relates to a method of improving the aesthetic appearance of skin by reducing and/or masking one or more of wrinkles, fine lines, pores, skin imperfections, and skin topology wherein said method comprises the step of applying to the areas of skin to be treated an effective amount of the cosmetic composition of the present invention.

The methods of use for the cosmetic composition disclosed and claimed herein concern the improvement in the aesthetic appearance of skin and include but are not limited to methods of masking one or more of wrinkles, fine lines, pores, skin imperfections, especially in the facial, neck or on or around the lip areas.

Facial lines and wrinkles can be present anywhere on the face and occur most frequently on the lips and in the eye area. However, it is understood by those skilled in the art that the composition can be applied to any part of the body where a blurring effect is desired such as to reduce wrinkles, fine lines, pores, and skin imperfections.

In a fourth aspect of the invention, the present invention relates to the use of the skin care cosmetic composition of the present invention for the treatment of signs of skin aging and/or skin imperfections.

In order to facilitate the understanding of the present invention, the meaning of some terms and phrases used in the context of the present invention is included herein.

In the context of the present invention, "skin" is understood to mean a layer consisting of the uppermost layer or the stratum corneum to the lowest layer or subcutaneous tissue (both including the end points). These layers are composed of different types of cells, such as keratinocytes, fibroblasts, melanocytes and/or adipocytes.

"Skin care" in the context of the present invention encompasses signs of preventing aging and/or photoaging.

In the context of the present invention, the term "aging" refers to changes in the skin as a function of age (time aging) or by exposure to the sun (photoaging) or exposure to environmental substances such as tobacco smoke, cold or wind extreme weather conditions, Changes in chemical contaminants or contaminants, and including all externally visible and/or perceptible changes by touch, especially for example and without limitation, development of discontinuities on the skin such as wrinkles, fine lines, expression lines, stretch marks , wrinkles, irregular or rough, increased pore size, loss of hydration, loss of elasticity, loss of firmness, loss of smoothness, loss of ability to recover from deformation, loss of resilience, sagging of the skin especially such as sagging cheeks, bags under the eyes appearance or double chin appears, skin tone changes such as spots, redness, eye bags or hyperpigmentation areas such as age spots or freckles, abnormal differentiation, hyperkeratosis, elastic tissue degeneration, keratosis, hair loss, orange peel skin, collagen Structural loss and other histological changes in the stratum corneum, dermis, epidermis, vascular system (eg spider veins or capillaries). Other histological changes in those tissues that are dilated or close to the skin.

The term "photoaging" will bring together a set of processes that cause skin premature aging due to prolonged exposure of the skin to ultraviolet radiation, and which exhibit the same physical characteristics as aging, such as and without limitation, relaxation, sagging, color change, or pigmentation. Irregular, abnormal and/or excessive keratinization. Several environmental factors such as exposure to tobacco smoke, exposure to pollution, and climatic conditions such as cold and/or wind also promote skin aging.

### DESCRIPTION OF EMBODIMENTS

### Example 1. Method of manufacturing the polymeric nanocapsules of the cosmetic composition of the present invention

A skilled person is aware of suitable apparatus and equipment for carrying out the process in accordance with the present invention and will select the suitable system based on his professional experience, so that no further extensive details need to be given here.

In this example the nanocapsules obtained have the following structure: the outer layer is made of sodium hyaluronate, the intermediate layer is made of a polycaprolactone, the inner layer is made of chitosan and the cosmetic active ingredient is acetylhexapeptide-8.

First, nanoparticles (NPs) of polycaprolactone (PCL) were prepared by means of a Water1/Oil/Water2 double emulsion-solvent evaporation method.

Initially, 5 mL of dichloromethane (DCM) with 0.68 g of polycaprolactone (PCL) (organic phase) was added to 1,25 mL of 0.2 g of acetylhexapeptide-8 and 0.1 g of sodium hyaluronate (HA) (extra low molecular weight) water solution (water phase 1) and then emulsified with by a homogenizer (UltraTurrax) at a rate of 16000 rpm for 15 min. The primary emulsion was poured into 50 mL of 1% PVA water solution (water phase 2) and it was emulsionated by ultrasonication for 10 minutes to form a double emulsion. Then, this solution was stirred till the organic solvent was totally evaporated.

The particle size and zeta potential of capsules was determined using Dynamic Light Scattering analyzer (DLS) (Malvern NanoZS, UK) and PCL NPs showed a narrow distribution, a particle size of diameters average of around 240 nm and zeta potential of -10 mV.

Subsequently, the functionalization of PCL NPs with HA (low molecular weight) was obtained via layer-by-layer method. It is no possible a direct coating of HA over PCL as both HA and PCL have negative surface charge, resulting in charge repulsion. Therefore, to coat NPs with HA, an intermediate layer of chitosan having positive charge was coated in between PCL and HA. For this purpose, PCL nanoparticles were added drop wise into a solution of 0.005 g of chitosan in a ratio of 1:2, with continuous stirring for 2 h. The zeta potential of the resulting solution was determined to confirm the coating, showing an average of around 8 mV.

After charge reversal, the prepared PCL-Chi, i.e. chitosan coated nanoparticles, were added to 0.01g of HA solution in the ratio of 1:3 to obtain PCL-Chi-HA nanocapsules. Zeta potential and particle size of the prepared sample was determined as -19 mV and 300 nm, respectively.

### Example 2. Skin penetration of the polymeric nanocapsules (Acetylhexapeptide-8@PCL-Chi-HA) of the cosmetic composition of the present invention

Initially, a comparison was made between the penetration capacity of the encapsulation of the active ingredient acetylhexapeptide-8 in the polymeric nanocapsule of the present invention (Acetylhexapeptide-8@PCL-Chi-HA, obtained in example 1) vs free acetylhexapeptide-8.

Cutaneous penetration of free acetylhexapeptide-8 and encapsulated acetylhexapeptide-8 (nanocapsule of the present invention) was determined by means Franz cells diffusion. The pig skin samples were used to carry out the experiment, cut into circles 2.5 cm in diameter. The skin was assembled on the receptor compartment of a Franz diffusion cell system with the stratum corneum facing up towards the donor compartment. As a solution for the receptor, 18 ml of phosphate buffered saline (PBS) pH 7.4 stirred by a magnetic rod at 700 rpm were utilized. A 25 µl aliquot of each of the assay formulations was applied on the pig skin. The Franz diffusion cells were kept at 32°C, and after 20 h of incubation, the receptor solution was analyzed by HPLC (I=220 nm) to quantify the amount of acetylhexapeptide-8 was penetrated the skin. Triplicates of the assay were done.

HPLC analysis was performed utilizing a Waters 996 instrument with diode array detector equipped with Waters 2695 separations module and Millenium software; a C18 4.6x150 mm 5 µm reversed phase column by Waters was used. UV detection was realized at 220 nm through linear gradient from 5 to 100% of acetonitrile (+0,036% TFA) in water at 1.0 mL/min of flow for 8 minutes. Under this condition the retention time of acetylhexapeptide-8 was 6.5 minutes.

The quantification of acetylhexapeptide-8 in the fluid (PBS) of the receptor chamber was evaluated through HPLC at 20 hours after application of 25 µL of the two solutions on the pig skin.

The results are shown in the table 1. As can be seen, the penetration of the acetylhexapeptide-8 when it is encapsulated is significantly higher than the free one.

**Table 1. Comparison of the % penetration of encapsulated Acetylhexapeptide-8 vs free Acetylhexapeptide-8.**

| **Time (h)** | **% Penetration** | |
|---|---|---|
| | **Free Acetylhexapeptide-8** | **Encapsulated Acetylhexapeptide-8 (Present invention)** |
| 20 | 14 | 60 |

### Example 3. Study of the neurotransmitter release inhibitory activity of the polymeric nanocapsules (Acetylhexapeptide-8-HA@PCL-Chi-HA) of the cosmetic composition of the present invention

A study to evaluate the increase of the neurotransmitter release inhibitory activity of the nanocapsules of the present invention (Acetylhexapeptide-8-HA@PCL-Chi-HA) was carried out.

In this example the nanocapsules obtained have the following structure: the outer layer is made of sodium hyaluronate, the intermediate layer is made of a polycaprolactone, the inner layer is made of chitosan and the cosmetic active ingredients are acetylhexapeptide-8 and sodium hyaluronate.

Chromaffin cell cultures were prepared from bovine adrenal glands by collagenase digestion and then separated from debris and erythrocytes by centrifugation. Briefly, the cells were maintained in monolayer cultures and used between the third and sixth day after plating. All the experiments were carried out at 37°C. Secreted neurotransmitter [³H]noradrenaline was determined in cells permeabilized with digitonin. Neurotransmitter (catecholamines) release was stimulated by addition of 10 µM) Ca²⁺.

Acetylhexapeptide-8 peptide mimics the action of botulinum neurotoxins in inhibiting the neurotransmitters release that causes muscle contraction. Consequently, a local relaxation of the musculature is observed, which is associated with a decrease in wrinkles.

To evaluate the enhancement of this activity of encapsulated Acetylhexapeptide-8, the inhibitory capacity on Ca²⁺-induced catecholamine release was measured in digitonin-permeabilized chromaffin cells culture.

In particular, the activity of the free peptide free (acetylhexapeptide-8), the peptide encapsulated in Policaprolactone-Chitosan (Acetylhexapeptide-8@PCL-Chi) and the peptide encapsulated with sodium hyaluronate (HA) in PCL-Chi-HA (Acetylhexapeptide-8-HA@PCL-Chi-HA) (present invention) were compared.

As illustrated in the table 3, detergent-permeabilized chromaffin cells released catecholamines in response to an increase in intracellular Ca²⁺ (control).

**Table 3. Cathecolamine release (%)**

| **Sample** | **Cathecolamine release (%)** |
|---|---|
| **Control** | 100 |
| **Free Acetylhexapeptide-8** | 80 |
| **Acetylhexapeptide-8@PCL-Chi** | 54 |
| **Present invention (Acetylhexapeptide-8-HA@PCL-Chi-HA)** | 52 |

When the chromaffin cells were incubated with free peptide, a 20% decrease in total catecholamine exocytosis was observed. Otherwise, Acetylhexapeptide-8 loaded-PCL-Chi inhibited by 46% the release of neurotransmitters, showing an increase in inhibitory activity, due both to a higher penetration of the peptide and to a lower degradation of the encapsulated peptide. No changes in peptide activity were observed in the presence of sodium hyaluronate.

### Example 4. Moisturizing efficacy of the polymeric nanocapsules (Acetylhexapeptide-8-HA@PCL-Chi-HA) of the cosmetic composition of the present invention

The transpidermal water loss (TEWL) study by corneometry was performed to compare the moisturizing activity of the composition of the present invention and its main components.

The assay was carried out on 20 women between the ages 45 and 50 years. None of the patients had suffered or had any medical history or relevant skin pathologies.

The volunteers were divided into four groups. Daily, for 7 days, the assigned sample was applied to one of the forearms of each group (group 1 sample with free sodium hyaluronate, group 2 sample with free Acetylhexapeptide-8, group 3 sample with free sodium hyaluronate and Acetylhexapeptide-8 and group 4 sample with complete encapsulation (Acetylhexapeptide-8, sodium hyaluronate, polycaprolactone and chitosan). The opposite forearm of each volunteer was the control.

Measurements were taken 2 hours and 7 days after the first application. Epidermal capacitance was evaluated using a CM 825 Corneometer, which allows quantification of surface humidity variations. Measurements were taken in an air-conditioned room (temperature 23°C, relative humidity 34.5%).

Corneometry consists of evaluating the capacitance of a dielectric medium. Small variations in the dielectric constant of the skin surface are related to skin hydration, so it is a widely implemented and highly accurate method, detecting minimal variations in the level of skin hydration. The measurement provides information on the hydration of the upper layers of the skin (10-20 µm) without interfering with its lower layers, by measuring transepidermal water loss (TEWL).

This trial compared the moisturizing capacity of free HA, free Acetylhexapeptide-8, free HA+Acetylhexapeptide-8 and the composition of the present invention (Acetylhexapeptide-8-HA@PCL-Chi-HA) by measuring the skin loss of water on the forearm of the volunteers analyzed. The patients were separated into 4 groups and each group was given the corresponding sample for 7 days. Two measurements were taken.

The first at two hours after the first application, as shown in the table 4, except for the application of the free peptide, in the rest of the tests after two hours of the first application a decrease in water loss was detected, being higher in the group that used the suspension with the complete encapsulation system.

In the second measurement at 7 days, a high inhibition of TEWL was observed in the application of the complete system sample (Acetylhexapeptide-8-HA@PCL-Chi-HA), indicating a higher accumulation in the skin of sodium hyaluronate due to its sustained release.

**Table 4. Transepidermal water loss (TEWL) after 2 hours and 7 days**

| **Samples** | **Tewl (g/m²h)** | |
|---|---|---|
| | **After 2 hours** | **After 7 days** |
| **Control** | 16 | 17 |
| **Free HA** | 12 | 9.8 |
| **Free Acetylhexapeptide-8** | 15.8 | 16.5 |
| **Free Acetylhexapeptide-8+HA** | 11 | 12 |
| **Acetylhexapeptide-8-HA@PCL-Chi-HA (Present invention)** | 9 | 4.4 |

### Example 5. Anti-wrinkle efficacy of the polymeric nanocapsules (Acetylhexapeptide-8-HA@PCL-Chi-HA) of the cosmetic composition of the present invention

The clinical study was conducted on 40 women with facial wrinkles between 50 and 60 years of age. All volunteers were healthy and without any systemic or skin disease.

Wrinkles were measured using a topographic skin measurement device, a Bio 3D Structure Light Scanner technology, which performs a 3D reconstruction of the image in silicone (ANTERA 3D^{®} and software (Miravex, Ireland).

The patients were divided into two groups, which applied the assigned O/W emulsions twice a day for two months. One group (1) applied a free Acetylhexapeptide-8 emulsion on one side of the preorbital area and an emulsion with Acetylhexapeptide-8 encapsulated in PCL and chitosan on the other side. The second group applied an O/W emulsion with free HA on one area of a preorbital area and another of the cosmetic composition of the present invention (Acetylhexapeptide-8, HA, PCL and chitosan) on the contralateral side. All participants were asked not to use any cosmetics containing ingredients that could interfere with the wrinkle status of the skin during the study period, nor were they subjected to exposure to direct sunlight.

Before the first application, at 4 and 8 weeks of use, silicone impressions were taken and processed by confocal laser scanning microscopy to evaluate the progress of wrinkles. The maximum depth values within the analysis area were taken as representative values of wrinkles, and the percentage reduction of these values before and after application were calculated as the percentage improvement of skin wrinkles.

In this study, measurements of the main roughness parameters such as mean roughness volume and area affected by roughness were obtained. The average roughness volume is the average (three-dimensional) dimension of the depression of the cutaneous microrelief measured in mm³. The area affected by roughness is the roughness portion of the measured skin area (two-dimensional, measured in mm²). The improvement was calculated as the percentage change between the images taken before the treatments and two months after the final treatment.

Reflection mode confocal microscopy and three-dimensional analysis were used to evaluate the different parameters.

Acetylhexapeptide-8, applied topically through cosmetic creams, is deposited in the superficial layers of the skin where it acts as an anti-wrinkle expression wrinkle reducer.

The clinical study execution allowed to compare the anti-wrinkle efficacy of free Acetylhexapeptide-8, free HA, Acetylhexapeptide-8 loaded-PCL-Chi (Acetylhexapeptide-8@PCL-Chi) and the cosmetic composition of the present invention (Acetylhexapeptide-8-HA@PCL-Chi-HA).

For this purpose, the 40 female volunteers were divided into two groups, and each group applied twice daily for 2 months two assigned emulsions, one to each eye in the same preorbital area. Before treatment, at 4 and 8 weeks, the decrease in wrinkles was quantified by 3D images analyzed by confocal microscopy.

As shown in the table 5, the application of free Acetylhexapeptide-8 decreased by 20% the wrinkle formation after 4 weeks and 30% at the end of the treatment. The effect was increased in the encapsulated version (Acetylhexapeptide-8@PCL-Chi), due to a higher bioavailability of the peptide which penetrates more into the skin when it is encapsulated and is more protected against degradation by cellular enzymes. The most surprising result was seen in the complete version (Acetylhexapeptide-8-HA@PCL-Chi-HA), where an increase in anti-wrinkle activity was observed with respect to Acetylhexapeptide-8@PCL-Chi, especially at 8 weeks.

The result was unexpected, considering that sodium hyaluronate has no anti-wrinkle effect, so it was deduced that there is a synergistic effect between the components that enhances the activity of the product.

**Table 5. Wrinkles reduction (%)**

| **Samples** | **Wrinkles reduction (%)** | |
|---|---|---|
| | **4 wrinkles** | **8 wrinkles** |
| **Free Acetylhexapeptide-8** | 20.5 | 30 |
| **Argirelina@PCL-Chi** | 30.2 | 35 |
| **Free HA** | 2 | 3 |
| **Acetylhexapeptide-8-HA@PCL-Chi-HA (Present invention)** | 40.5* | 60*^{#} |

| | | |
|---|---|---|
| *p < 0,05 vs Acetylhexapeptide-8, # p < 0,05 vs 4 weeks | | |

The same synergistic effect was the cause of the unexpected improvement in roughness reduction when the composition of the present invention was applied compared to free Acetylhexapeptide-8 and Acetylhexapeptide-8@PCL-Chi. Also in this case, it was excluded that the increase was due to hyaluronate, since, as shown in the table 6, it does not have this effect.

**Table 6. Reduction of roughness parameters at 8 weeks**

| **Samples** | **Reduction of roughness parameters at 8 weeks** | |
|---|---|---|
| | **Roughness volume decrease (%)** | **Decrease area affected by roughness (%)** |
| **Free Acetylhexapeptide-8** | 35 | 31.2 |
| **Acetylhexapeptide-8@PCL-Chi** | 37.4 | 34.5 |
| **Free HA** | 1 | 2 |
| **Acetylhexapeptide-8-HA@PCL-Chi-HA (Present invention)** | 55.7* | 58* |

| | | |
|---|---|---|
| *p < 0,05 vs Acetylhexapeptide-8 | | |

## Claims

1. A skin care cosmetic composition comprising polymeric nanocapsules encapsulating at least one cosmetic active ingredient, wherein the polymeric nanocapsules comprising:
- an outer layer made of sodium hyaluronate or fucoidan;
- an intermediate layer made of a polysaccharide; and
- an inner layer made of a biodegradable polymer; and
wherein the cosmetic active ingredient is selected from the group consisting of acetylhexapeptide-8, sodium hyaluronate, fucoidan or a mixture thereof.

2. The skin care cosmetic composition according to claim 1, wherein the polymeric nanocapsules comprises 50% to 68.5% by weight of biodegradable polymer, 30% to 45% by weight of active ingredient, 1% to 3% by weight of sodium hyaluronate or fucoidan in the outer layer and 0,5 to 2% by weight of polysaccharide, based on the total weight of the polymeric nanocapsules.

3. The skin care cosmetic composition according to claim 1 or 2, wherein the polysaccharide is selected from chitosan or dextran.

4. The skin care cosmetic composition according to any one of claims 1 to 3, wherein the biodegradable polymer is selected from the group consisting of policaprolactone (PCL), poly(lactic-co-glycolic acid) (PLGA), poly-lactic acid (PLA) or carrageenan.

5. The skin care cosmetic composition according to any one of claims 1 to 4, wherein the polymeric nanocapsules comprises 50% to 68.5% by weight of polycaprolactone, 20% to 25% by weight of acetylhexapeptide-8, 10% to 20% by weight of sodium hyaluronate (as active ingredient), 1% to 3% by weight of sodium hyaluronate (in the outer layer) and 0,5 to 2% by weight of chitosan,, based on the total weight of the polymeric nanocapsules.

6. The skin care cosmetic composition according to any one of claims 1 to 5, wherein the composition further comprises one or more cosmetically acceptable excipients.

7. The skin care cosmetic composition according to any one of claims 1 to 6, wherein the composition is formulated as an emulsion, a cream, a lotion, a solution, a gel, an ointment or a serum.

8. Method of manufacturing the polymeric nanocapsules of the skin care cosmetic composition according to claim 1, comprising the following steps:
a) adding a biodegradable polymer together with at least one cosmetic active ingredient wherein the cosmetic active ingredient is selected from the group consisting of acetylhexapeptide-8, sodium hyaluronate, fucoidan or a mixture thereof in the presence of an organic solvent to create a double emulsion;
b) stirring the emulsion obtained in step a) until complete evaporation of the organic solvent to obtain nanoparticles;
c) dropping the nanoparticles obtained in step b) into a solution of polysaccharide with continuous stirring; and
d) adding the polysaccharide-coated nanoparticles obtained in step c) to a solution of sodium hyaluronate or fucoidan to obtain the polymeric nanocapsules.

9. The method according to claim 8, wherein the ratio of nanoparticles and the solution of polysaccharide in step c) is 1:2.

10. The method according to any one of claims 8 to 9, wherein the ratio of polysaccharide-coated nanoparticles and the solution of sodium hyaluronate in step d) is 1:3.

11. The method according to any one of claims 8 to 10, wherein the polysaccharide is selected from chitosan or dextran.

12. The method according to claim 8, wherein the biodegradable polymer is selected from the group consisting of policaprolactone (PCL), poly(lactic-co-glycolic acid) (PLGA), poly-lactic acid (PLA) or carrageenan.

13. The method according to any one of claims 8 to 12, wherein the continuous stirring in step c) is for 2 h.

14. A method of improving the aesthetic appearance of skin by reducing and/or masking one or more of wrinkles, fine lines, pores, skin imperfections, and skin topology wherein said method comprises the step of applying to the areas of skin to be treated an effective amount of the skin care cosmetic composition according to any one of claims 1 to 7.

15. Use of the skin care cosmetic composition according to any one of claims 1 to 7 for the treatment of signs of skin aging and/or skin imperfections.
